# EUROPEAN PATENT APPLICATION

(11) **EP 2 638 969 A2**
(43) Date of publication of application: **18.09.2013**
(21) Application number: 13158753.7
(22) Date of filing: 12.03.2013
(51) Int. Cl.: B05B 1/02, A61M 11/00

(54) **Applicator spray nozzles with pressure relief**

(30) Priority: 13.03.2012 US 201261610239 P; 26.11.2012 US 201261729839 P; 06.03.2013 US 201313786737
(71) Applicant: Nordson Corporation, Westlake, OH 44145 (US)
(72) Inventor: Hoogenakker, Jon E., Inver Grove Heights, Minnesota 55077 (US); Lou, Huadong, Maple Plain, Minnesota 55108 (US); Robb, Bradley D., Maple Plain, Minnesota 55359 (US)
(74) Representative: Eisenführ, Speiser & Partner

(57) **Abstract**

A spray nozzle for applying a medicinal agent to a tissue. The spray nozzle comprising a tip housing and a valve. The tip housing has a distal end, a proximal end, and a lumen that extends between the ends and defines a lengthwise central axis. The valve has an elongated body that extends through the lumen of the tip housing, along the lengthwise central axis. The valve, being configured to slide within the lumen of the tip housing, in an extended position with respect therewith is configured to disperse the medicinal agent toward the tissue. When the valve is in the retracted position with respect to the tip housing, the medicinal agent is vented away from the tissue.

## Description

### Cross-Reference to Related Applications

This application claims the priority of U.S. Provisional Patent Application Serial Nos. 61/610,239, filed on March 13, 2012 (pending) and 61/729,839, filed November 26, 2012 (pending), the disclosures of which are incorporated by reference herein in their entirety.

### Technical Field

The present invention relates generally to spray applicators and, more particularly, to applicators configured to deliver therapeutic aerosols to a surgical site of a patient.

### Background

Intra-operative problems encountered by physicians often include management and prevention of post-operative pain, infection, tissue adhesion, and tumor formation. Numerous products exist on the market to address these problems by improving the surgical experience and patient outcomes. Among these products are suction and irrigation wands that are used for flushing tissue sites with sterile water or saline and removing blood and other fluids from the surgical site. Spray applicators may be used to deliver therapeutic aerosols, fluidized powder products, and/or gas streams to anatomical surfaces within a surgical site of a patient. The therapeutic aerosols may be applied to an open anatomical surface or within an artificially created surgical site, such as during minimally invasive surgery. The aerosol formulations may be delivered at any time before, during, or after a surgical procedure.

However, while applying the therapeutic aerosol by the spray applicator during a topical or laparoscopic procedure, there exists a risk that the applicator tip may come into contact with patient tissue comprising the surgical site. If tissue contact closes off the tip from which the medicine is dispensed, then pressure may build up within the applicator. Still worse, the force of the pressurized gas, applied at extremely close proximity, may drive fluids (including gases) into a cut blood vessel, causing pressure injury or embolism, or otherwise damage tissues already inflamed and distressed by the surgical procedure. Therefore, there exists a need for spray applicators and methods of use that reduce the adverse affects of inadvertent contact between the applicator tip and the surgical site.

### Summary of the Invention

In accordance with one embodiment of the present invention a spray nozzle for applying a medicinal agent to a tissue includes a tip housing and a valve. The tip housing is positioned on a distal end of a tubing and the valve is movably positioned within the tip housing. The valve is configured to move from an extended position, in which the medicinal agent is directed away from the tip housing toward the tissue, and a retracted position, in which the medicinal agent is vented away from the tissue.

According to one embodiment of the present invention, a spray nozzle for applying a medicinal agent to a tissue includes a tip housing and a valve. The tip housing has a distal end, a proximal end, and a lumen that extends between the ends and defines a lengthwise central axis. The valve has an elongated body that extends through the lumen of the tip housing, along the lengthwise central axis, and slides with respect thereto between an extended position and a retracted position. A spin chamber at the distal end of the elongated body of the valve is configured to mix the medicinal agent. An insert, positioned within the lumen of the valve and proximate to the spin chamber, includes a gas bubble that receives the mixed medicinal agent and disperses the same. A plurality of relief vents, which are formed on an outer surface of the elongated body of the valve, is configured to be in fluid communication with the lumen of the tip housing when the valve is in the retracted position. When the valve is in the extended position with respect to the tip housing, the spray nozzle is configured to disperse the medicinal agent via the spin chamber and the insert toward the tissue; when the valve is in the retracted position with respect to the tip housing, the spray nozzle is configured to vent the medicinal agent away from the tissue via the plurality of relief vents. An additional feature is provided for mixing the medicinal agent at the tip. In this regard, mixing structure such as one or more generally spiral fins are provided in the tip housing. These may be used, for example, to mix a powder agent with a gas prior to dispersion. In another aspect, the tip housing may include a connector element, and the distal end of the applicator tubing may include a mating connector element for allowing a user to selectively couple the tip housing and valve to the applicator tubing. For example, these connector elements may be mating threads.

Still other embodiments of the present invention are directed to a spray applicator comprising a spray nozzle and a tubing. The spray nozzle tissue includes a tip housing and a valve. The tip housing is positioned on a distal end of a tubing and the valve is movably positioned within the tip housing. The valve is configured to move with respect to the tip housing between an extended position and a retracted position. The tubing is operably coupled to the proximal end of the tip housing. When the valve is in the extended position with respect to the tip housing, the spray nozzle is configured to disperse the medicinal agent toward the tissue; when the valve is in the retracted position with respect to the tip housing, the spray nozzle is configured to vent the medicinal agent away from the tissue.

According to embodiments of the present invention, methods of applying one or more medicinal agents to a tissue using a spray nozzle include directing a medicinal agent toward the tissue while the valve is in the extended position and venting the medicinal agent when the valve is in the retracted position.

Another embodiment of the present invention is directed to a removal tool that is configured to remove a spray nozzle from a tubing. The removal tool includes a cover portion and a disengagement portion. The cover portion operably engages and supports the tip housing of the spray nozzle. While the disengagement portion releases the tip housing from the tubing, the cover portion is configured to move with respect to the disengagement portion such that the spray nozzle is withdrawn from the tubing.

### Brief Description of the Drawings

FIG. 1 is a side elevational view of a spray applicator having a spray nozzle in accordance with one embodiment of the present invention.

FIG. 2 is an exploded, perspective view of the spray nozzle of FIG. 1 having a tip housing and a valve in accordance with one embodiment of the present invention.

FIGS. 3A and 3B are cross-sectional views of the assembled spray nozzle of FIG. 2, the valve shown in an extended position and a retracted position, respectively, with respect to the tip housing.

FIG. 4A is a cross-sectional view of the assembled spray nozzle of FIG. 2 illustrating the valve in the extended position such that medicinal agent is directed away from the tip housing and toward a tissue.

FIG. 4B is a cross-sectional view of the assembled spray nozzle of FIG. 2 illustrating the valve in the retracted position such that the medicinal agent is vented away from the tissue.

FIGS. 5A-5C are cross-sectional views of the tip housing of FIG. 2 with valves of varying length and in accordance with other embodiments of the present invention.

FIG. 6A is an exploded, perspective view of a spray nozzle having the tip housing of FIG. 2 and a valve in accordance with another embodiment of the present invention.

FIG. 6B is a cross-sectional view of the assembled spray nozzle of FIG. 6A.

FIG. 7 is a perspective view of a tip housing in accordance with another embodiment of the present invention.

FIG. 8 is an exploded, side elevational view of a spray nozzle having a tip housing and a valve in accordance with another embodiment of the present invention.

FIGS. 9A and 9B are exploded, side elevational views of spray nozzles having tip housings and valves according to other embodiments of the present invention.

FIG. 10 is an assembled, side elevational view of the spray nozzle of FIG. 9B.

FIG. 11 is a cross-sectional view of the assembled spray nozzle of FIG. 10.

FIG. 12 is a cross-sectional view through a spin chamber of the spray nozzle and taken along the line 12-12 of FIG. 11.

FIG. 13A is a side elevational view of a spray chamber insert configured to be positioned within the valve and in accordance with one embodiment of the present invention.

FIG. 13B is a front end view of the insert shown in FIG. 13A.

FIG. 14 is a cross-sectional view of the spray nozzle of FIG. 10 with the valve in an extended position with respect to the tip housing such that the medicinal agent is directed away from the tip housing and toward a tissue.

FIG. 14A is an enlarged cross-sectional view of the spray nozzle of FIG. 14.

FIG. 14B is a cross-sectional view through the spin chamber of the spray nozzle and taken along the line 14B-14B of FIG. 14A.

FIG. 15 is a cross-sectional view of the spray nozzle of FIG. 10 with the valve in a retracted position with respect to the tip housing such that the medicinal agent is vented away from the tissue.

FIG. 15A is an enlarged cross-sectional view of the spray nozzle of FIG. 15.

FIG. 16A is a side elevational view of a spray chamber insert in accordance with another embodiment of the present invention.

FIG. 16B is a front end view of the insert shown in FIG. 16A.

FIG. 17 is a cross-sectional view of a spray nozzle in accordance with another embodiment of the present invention, in use with the insert of FIG. 16A, and in which the valve is in an extended position with respect to the tip housing such that the medicinal agent is directed away from the tip housing toward a tissue.

FIG. 17A is an enlarged cross-sectional view of the spray nozzle shown in FIG. 17.

FIG. 18A is a side-elevational view, in partial cross-section, of the spray nozzle of FIG. 10 and a spray nozzle removal tool according to one embodiment of the present invention coupled to the spray nozzle.

FIG. 18B is a side elevational view similar to FIG. 18A but with the spray nozzle removed from the tubing via the spay nozzle removal tool.

FIG. 19 is an exploded, perspective view of a spray nozzle having a tip housing and a valve in accordance with another embodiment of the present invention.

FIG. 20A is a cross-sectional view of the assembled spray nozzle of FIG. 19, with the valve shown in an extended position with respect to the tip housing.

FIG. 20B is a cross-sectional view of the assembled spray nozzle of FIG. 19, similar to FIG. 20A but the section being taken with the spray nozzle rotated 90° relative to Fig. 20A, and with the valve shown in a retracted position with respect to the tip housing.

FIGS. 21A and 21B are respective cross sectional views of the proximal end of a spray applicator constructed in accordance with another embodiment of the invention.

FIG. 22 is a cross sectional view similar to FIGS. 21A and 21B, but illustrating an alternative spacer design used between the inner and outer tubings.

FIG. 23 is a disassembled perspective view illustrating an alternative spray nozzle combining a valve feature with mixing structure.

FIG. 24A is a cross sectional view of the assembled spray nozzle of FIG. 23, and illustrating the valve in an extended or open position.

FIG. 24B is a cross sectional view similar to FIG. 24A, but illustrating the valve in a retracted or closed and venting position.

FIG. 25A is a disassembled cross sectional view showing an alternative spray nozzle and tip design.

FIG. 25B is a cross sectional view similar to FIG. 25A, but illustrating the spray nozzle assembled with a distal end of the spray applicator.

### Detailed Description

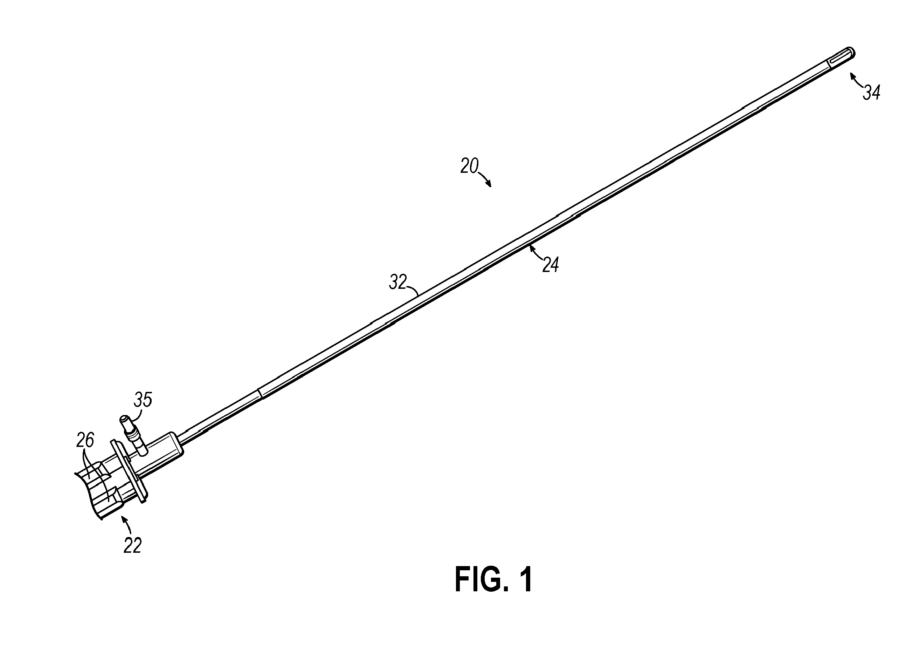

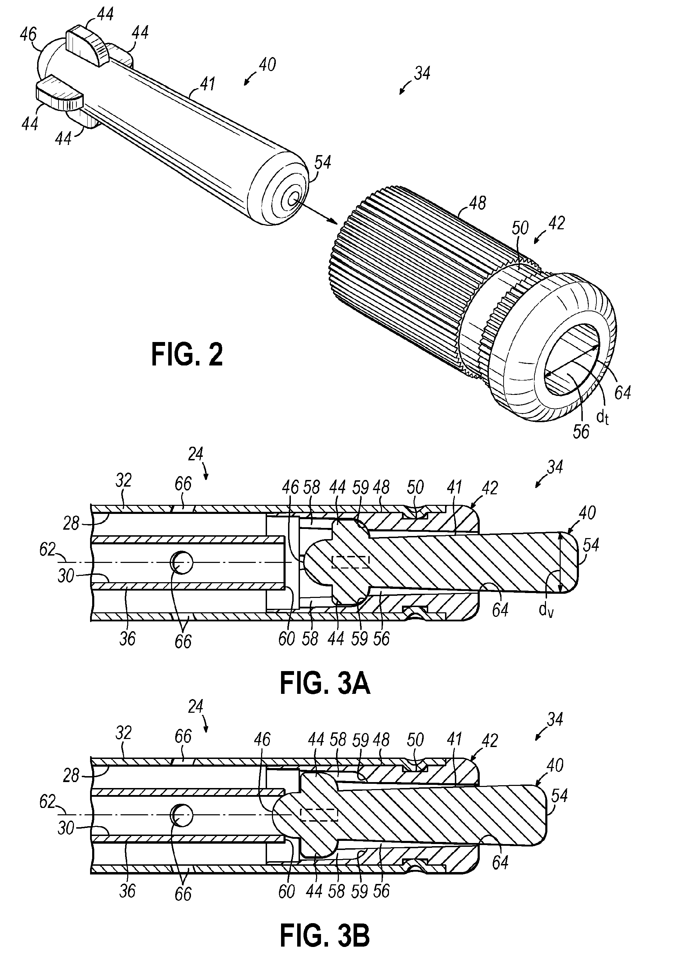

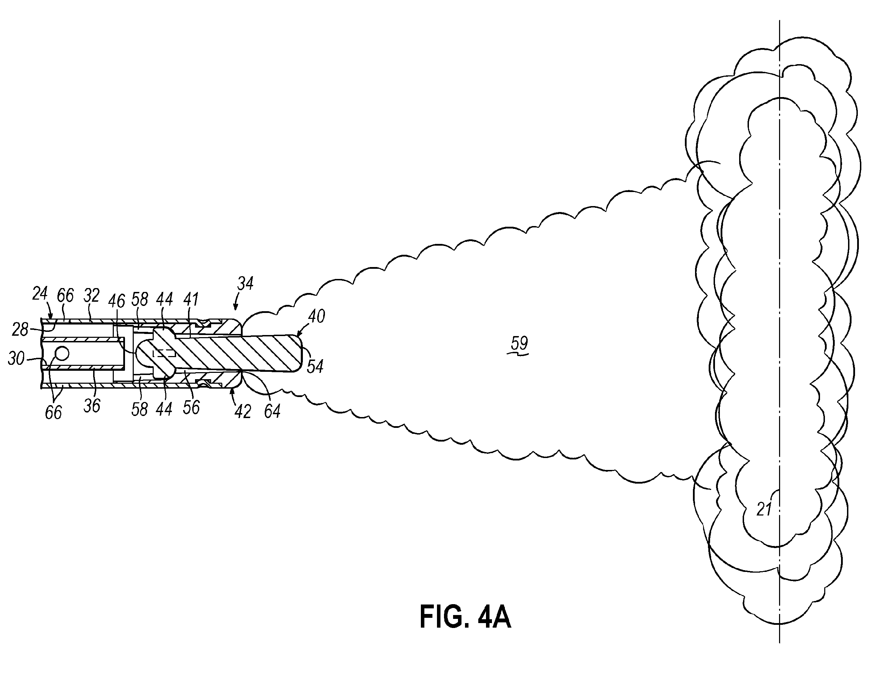

It will be appreciated that when referring to the drawings of the various embodiments illustrated and described herein, like reference numerals refer to like structure or elements shown in the figures. Therefore, with regard to various common features shown in different drawings, repeated discussion is not necessary, but the structure and function will be understood based on an earlier discussion of the structure or element. Turning now to the figures, and in particular to FIG. 1, a spray applicator 20 configured to apply a therapeutic aerosol spray onto a surgical site (represented by dashed line 21 of FIG. 4A) and in accordance with one embodiment of the present invention is shown. In the particular illustrative embodiment, the spray applicator 20 includes a proximal hub 22 having two parallel ports 26 for receiving a dual chamber syringe (not shown) and that may be utilized to transport one or more fluids into a multi-lumen tube 24; however, it would be readily understood that any number of ports may be included if so desired. The hub 22 includes passages (not shown) extending from the ports 26 and in fluid communication with the lumens 28, 30 (FIG. 3A) of the multi-lumen tube 24.

The multi-lumen tube 24, as shown in FIG. 3A, may comprise an outer tubing 32 providing the first lumen 28 extending from the hub 22 (FIG. 1), proximally, to a distally-positioned spray nozzle 34 and an inner tubing 36 providing the second lumen 30 extending from hub 22 (FIG. 1), proximally, to a distal position that is proximate to the spray nozzle 34. The outer tubing 32 may be constructed from rigid or compliant material, depending on the desired function of the application. The inner tubing 36 may be constructed from a hypotube or a flexible catheter material as would be known to those of ordinary skill in the art.

In use, the first lumen 28 may be configured to transfer a pressurized gas to the spray nozzle 34 from a pressurized container or a sterilized air system, which are commonly available at medical treatment facilities. This will provide a protective envelope of gas for the nozzle 34. A continuous flow of gas through the first lumen 28 will protect against condensation or other moisture building at the distal end of the nozzle 34 which would have negative effects on powder discharge. The air system may be coupled to the first lumen 28 via a side port 35 of the hub 22. Use of the air system may provide a constant overpressure to prevent contamination and/or plugging of the spray nozzle 34 between uses. The second lumen 30 may be configured to transfer a medicinal agent to the spray nozzle 34 from the dual chamber syringe (not shown) secured to the parallel ports 26 of the hub 22.

In FIGS. 2-3B, the spray nozzle 34 is shown in greater detail to include a valve 40 that is inserted into and in slidable relation with a tip housing 42. The valve 40 has an elongated body 41 that may be oblong- or harpoon-shaped with one or more blades 44 positioned near a proximal end 46 thereof. The distal end 54 is shaped to have a maximum width dimension, dᵥ, shown in FIG. 3A as a diameter offset from the distal edge of the valve 40, with a shape (i.e., radius of curvature, smoothness, and so forth) tapering longitudinally away from the maximum width dimension. The shape of the distal end 54 may be selected to provide a particular pattern of spray, e.g., the shape of the distal end 54 of the valve 40 may be used to direct the expelled aerosol to fill a wide conical shape or to focus centrally as the aerosol extends away from the spray nozzle 34.

The tip housing 42 may include a generally cylindrical outer surface 48 and, as shown in the instant embodiment, having an annular groove 50 such that the outer tubing 32 may be secured onto the tip housing 42, such as by crimping; although other methods may be used, some of which are described in detail below. The proximal end of the tip housing 42 may be configured to facilitate assembly to the outer tubing 32. Additionally, and as shown in FIG. 2, the distal end of the tip housing 42 may include an opening 64 having a width dimension (such as a diameter), dₜ, shape, and configuration to further direct the aerosol into a particular flow pattern. Generally, the width dimension of the opening 64 is approximately similar to, or slightly larger than, the maximum width dimension of the valve 40 such that dₜ ≥ dᵥ and to create a small clearance provided between the elongated body 41 of the valve 40 and the distal opening 64 of the tip housing 42.

Referring specifically now to FIGS. 3A and 3B, a lumen 56 extends through the tip housing 42 and is configured to receive the valve 40 therein such that the valve 40 is configured to slide with respect thereto. A portion of the inner wall comprising the lumen 56 may include a tapered guide portion 58 configured to receive the blades 44 of the valve 40 such that the blades 44 may rotate with respect to, and slide within, the tip housing 42. In that regard, the valve 40 slides between a distal taper edge 59 of the guide portion 58 and a positive stop 60, shown as the distal edge of the inner tubing 36. Therefore, the valve 40 may slide along a lengthwise central axis 62 of the multi-lumen tube 24 and the tip housing 42, between an extended position, shown in FIG. 3A, and a retracted position, shown in FIG. 3B.

In some embodiments, the blades 44 and the guide portion 58 may additionally or alternatively be configured to facilitate proper alignment of the valve 40 within the tip housing 42.

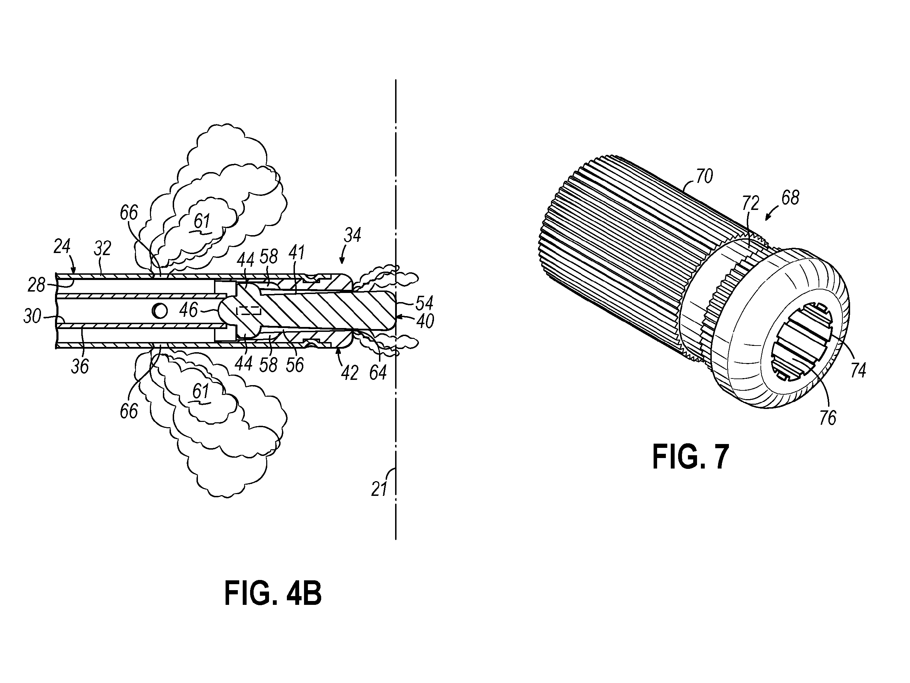

In use and with reference now to FIGS. 4A and 4B, the liquid or fluidized dry powdered product (such as with a medicinal agent) may be directed from the hub 22 (FIG. 1), through the inner and/or outer tubing 36, 32 to the spray nozzle 34. The fluids enter the lumen 56 of the tip housing 42 and flow between the blades 44 of the valve 40 toward the opening 64. The fluid flow between the blades 44 may be operable to cause the valve 40 to spin within the guide portion 58, which evens out the spray pattern and to mix the fluids so as to be effective for a selected purpose, such as stopping unwanted bleeding within the surgical site 21.

At the distal opening 64, and with the valve 40 being in the extended position as shown in FIG. 4A, the fluids are ejected as a dispersion 59 toward the tissue 21. The dispersion 59 expands as the fluid moves through the constricted area of the small clearance provided between the elongated body 41 of the valve 40 and the distal opening 64, e.g., from an area of higher pressure to an area of lower pressure, to cover a large surface area.

In FIG. 4B, if or when the spray nozzle 34 makes contact with the tissue 21, this contact creates a proximally-directed force that causes the valve 40 to move proximally within the tip housing 42 from the extended position, shown in FIG. 4A, to the retracted position as shown. With the valve 40 in the retracted position, the proximal end 46 of the valve 40 will be proximate to, or will at least partially enter into, the lumen 30 of the inner tube 36, thereby blocking and reducing fluid flow that may otherwise cause pressure injury or embolism. Furthermore, movement of the valve 40 into this retracted position brings the maximum width dimension, dᵥ (FIG. 3A), of the distal end 54 in closer proximity to the width dimension, dₜ (FIG. 2) of the tip housing 42. As dᵥ converges toward dₜ (dᵥ → dₜ), the small clearance provided between the elongated body 41 of the valve 40 and the distal opening 64 is reduced or closed. Fluid flow from the tip housing 42 is reduced, which causes pressure to build within the tip housing 42. Release of this increased pressure (as a vented powder 61) may be provided by one or more openings 66 within the outer tubing 32 and positioned proximal to the proximal end of the tip housing 42. Depending on the particular use of the spray nozzle 34, the vented powder 61 may be released into the abdomen cavity or surrounding atmosphere or, alternatively, may enter a surrounding tubing (not shown) in fluid communication with a vacuum line for disposal. The use of a vacuum line may be particularly useful when vented powder 61 may obscure the view through a laparoscopic camera or otherwise contaminate the surgical site 21.

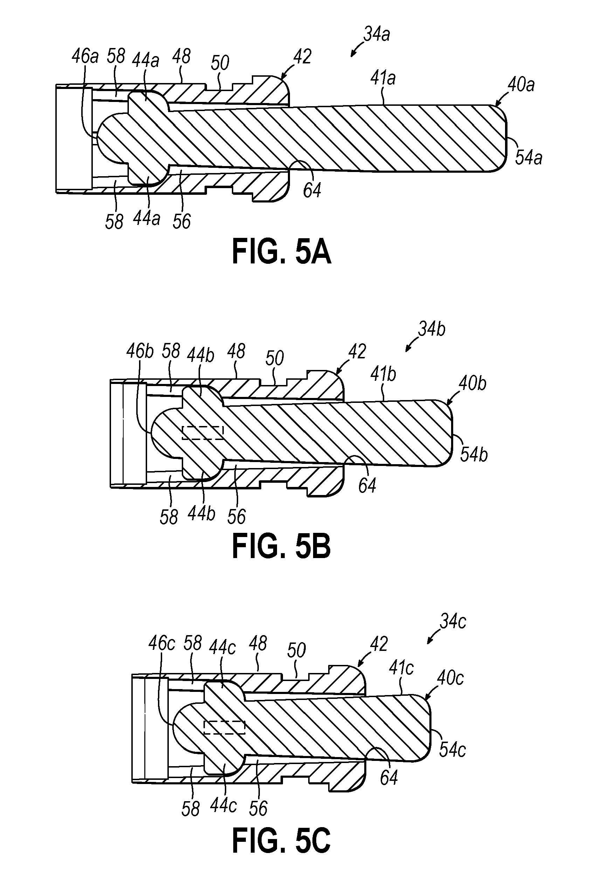

It would be thus appreciated from the teachings provided herein that selection of a shape for the valve 40 may be based, at least in part, on physician preference, the medical procedure, the medicinal agent being applied, and so forth. In that regard, the valve 40 may vary in length for use in particular procedures. As shown in FIGS. 5A-5C, spray nozzles 34a, 34b, 34c have the same tip housing 42 as FIG. 2, but with the elongated bodies 41a, 41b, 41c of the valves 40a, 40b, 40c vary in length, such as 10 mm, 5 mm, and 3 mm, respectively. However, these lengths are only exemplary in nature and these dimensions shall not be considered to be limiting.

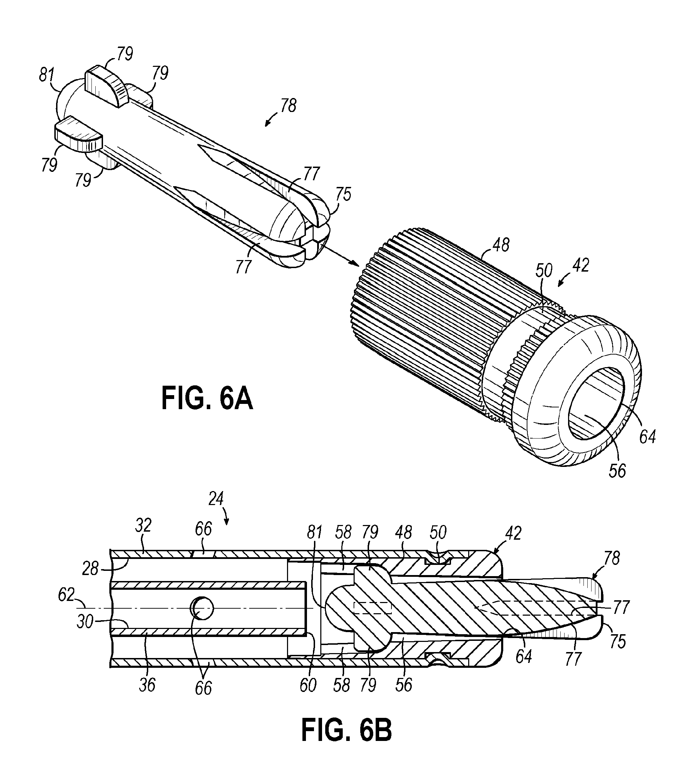

Yet, length of the valve 40 need not be the only variable feature. In FIGS. 6A and 6B, a valve 78 in accordance with another embodiment of the present invention is shown and includes a plurality of vanes 77 extending into a surface of the valve 78 and decrease in depth proximally from the distal end 75 toward the blades 79 at the proximal end 81. The depth, angle, shape, and number of the vanes 77 may vary to provide a particular shape to the expelled dispersion 59 (FIG. 4A).

In still other embodiments, such as is shown in FIG. 7, the tip housing 68 may also be altered to provide a particular shape of the dispersion 59 (FIG. 4A). According to the illustrative embodiment shown in FIG. 7, the tip housing 68 includes a body 70 with an outer groove 72 to receive the outer tubing 32 (FIG. 3A), and a textured lumen 76 extending therethrough from the distal opening 74. The texture of the lumen 76 may include grooves, as shown, or other patterns as desired to effectuate a particular fluid mixing pattern within the tip housing 68 and/or expulsion of the fluid therefrom.

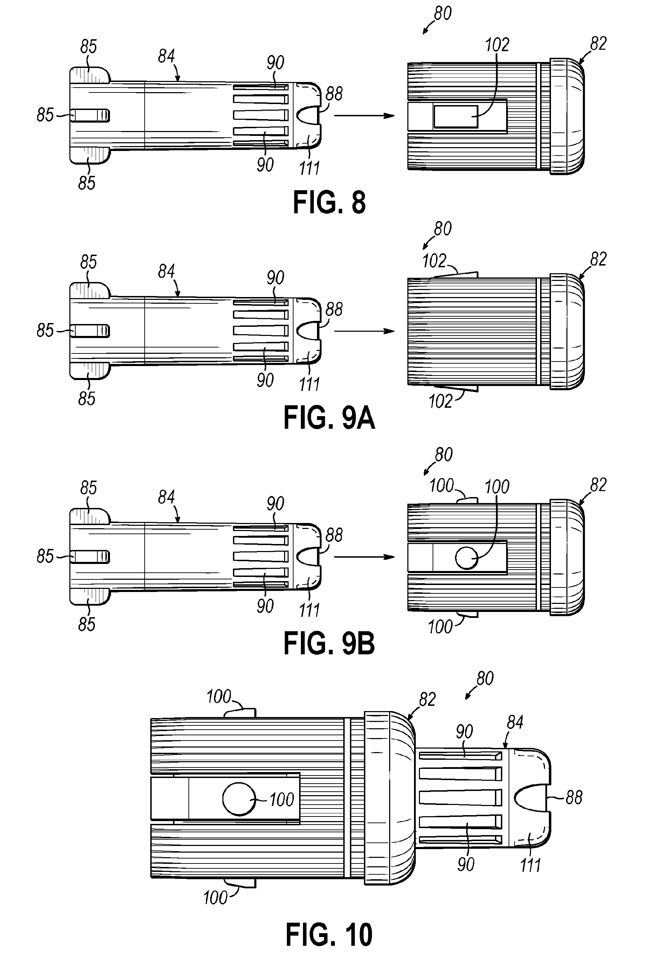

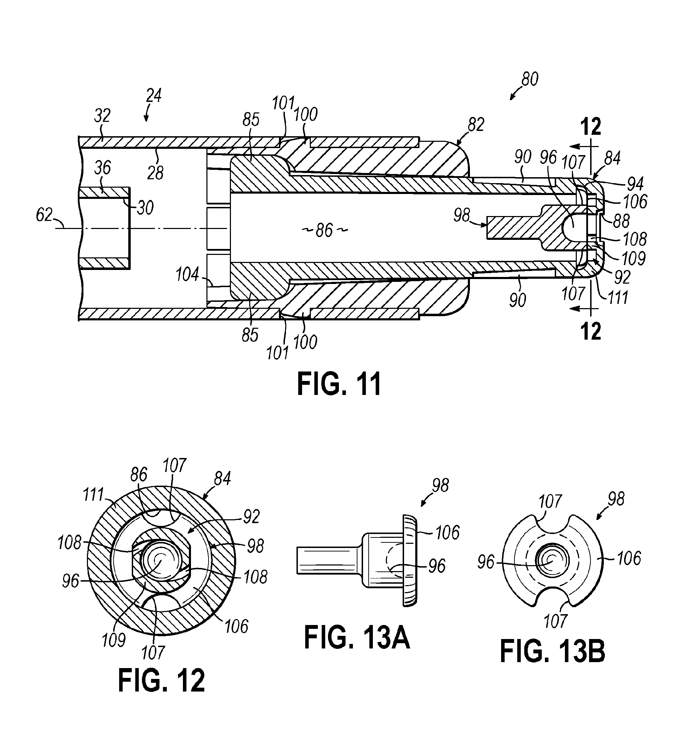

In still other embodiments, for example, as shown in FIGS. 8-9B, the outer groove 50 (FIG. 2) of the tip housing 82 may be replaced, or otherwise incorporated with, one or more snaps 100, as shown in FIG. 9B, or tabs 102, as shown in FIGS. 8 and 9A. The snaps 100 and/or tabs 102 are configured to be received by a space or recess 101 (FIG. 11) within the outer tubing 32 (FIG. 11).

FIG. 8 also illustrates a spray nozzle 80 in accordance with another embodiment of the present invention. The spray nozzle 80 includes a tip housing 82 and a valve 84 configured to be in slidable relation to the tip housing 82.

FIG. 11 illustrates the details of the valve 84 in greater detail as including a lumen 86 extending therethrough to a distal opening 88 and a plurality of relief vents 90 on an outer surface thereof. The relief vents 90, being positioned proximal to the distal opening 88 and arranged circumferentially about the valve 84, are tapered proximally in width and/or depth without forming fluid communication with the lumen 86 of the valve 84.

With reference to FIGS. 11-13B, the distal end of the valve 84 turns radially inwardly to form an inner wall 109 defining a spin chamber 92 between inner and outer walls 109, 111. The inner wall 109 includes a plurality of vanes 108 therethrough, which may be curved, arcuate, or otherwise angled for mixing the fluids during expulsion. A spin chamber insert 98 resides within the lumen 86 of the valve 84, proximate the spin chamber 92. A concave recess 96, in the form of a domed-shaped reflector, may be formed in a distal surface 106 of the spin chamber insert 98 to provide low friction relief for spinning fluids and powders entering from the plurality of vanes 108 prior to expulsion from the spray nozzle 80. The recess 96 will trap particles of powder to prevent blockage at the distal end of the valve 84. The size and concavity of the bubble 96 may be controlled to prevent collapse of the liquid or powdered vortex formed within the spin chamber 92. The spin chamber insert 98, while residing within a shelf 94 adjacent the lumen 86 of the valve 84, includes one or more cutouts 107 to allow fluid flow into the spin chamber 92 as described in detail below.

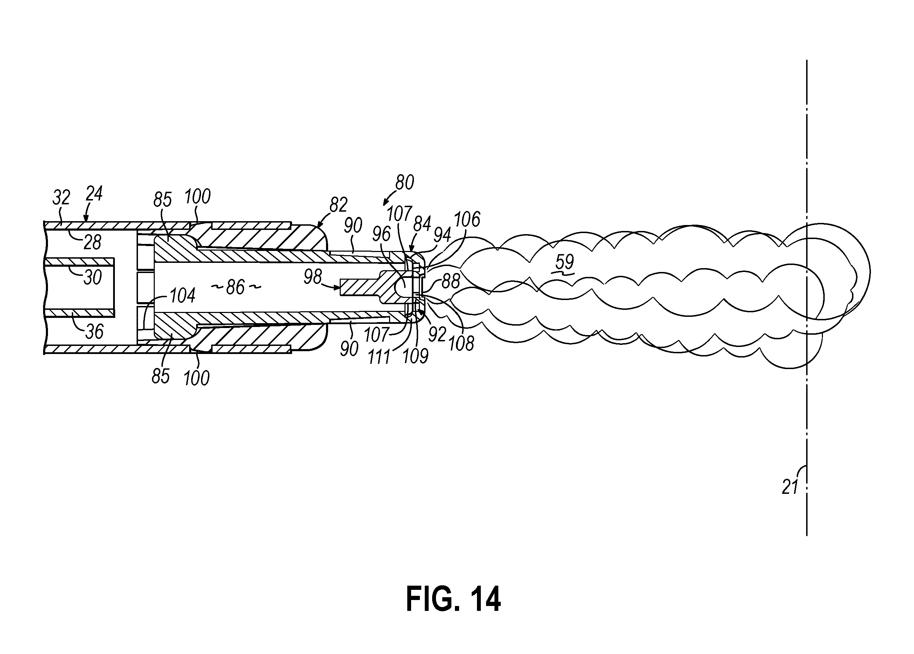

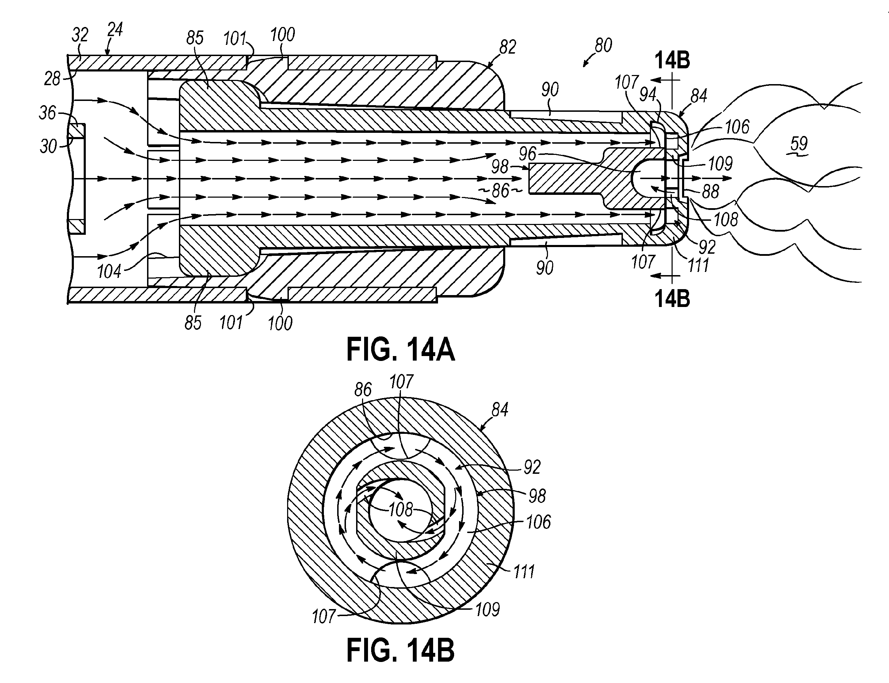

Use of the spray nozzle 80 is shown in FIGS. 14-15A. That is, during normal operation of the spray nozzle 80, shown in FIGS. 14 and 14A, the valve 84 is in an extended position with respect to the tip housing 82 such that fluid moves (indicated with arrows in FIG. 4A) along a first fluid path from the inner and/or outer tubes 36, 32, into the tip housing 82, into the lumen 86 of the valve 84, and outwardly from the distal opening 88. At the spin chamber 92, shown in FIG. 14B, the fluid flow (indicated with arrows) moves from the lumen 86 of the valve 84, between the inner and outer walls 109, 111 by way of the cutouts 107 of the insert 98. The fluid then flows through the vanes 108 of the inner surface 106 and into the bubble 96 where the fluid turns and is reflected to be expelled from the spray nozzle 80 as the dispersion 59.

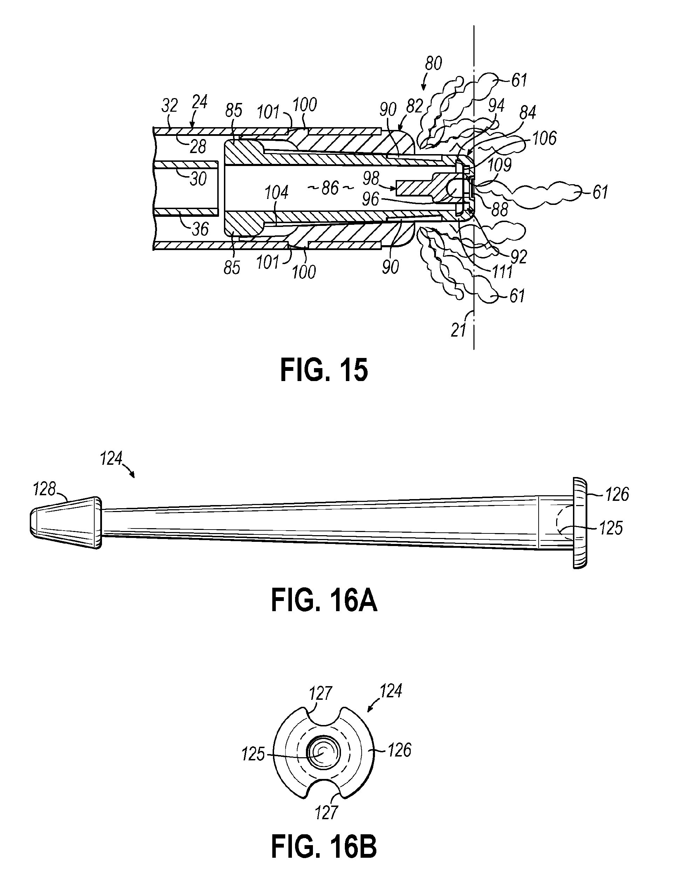

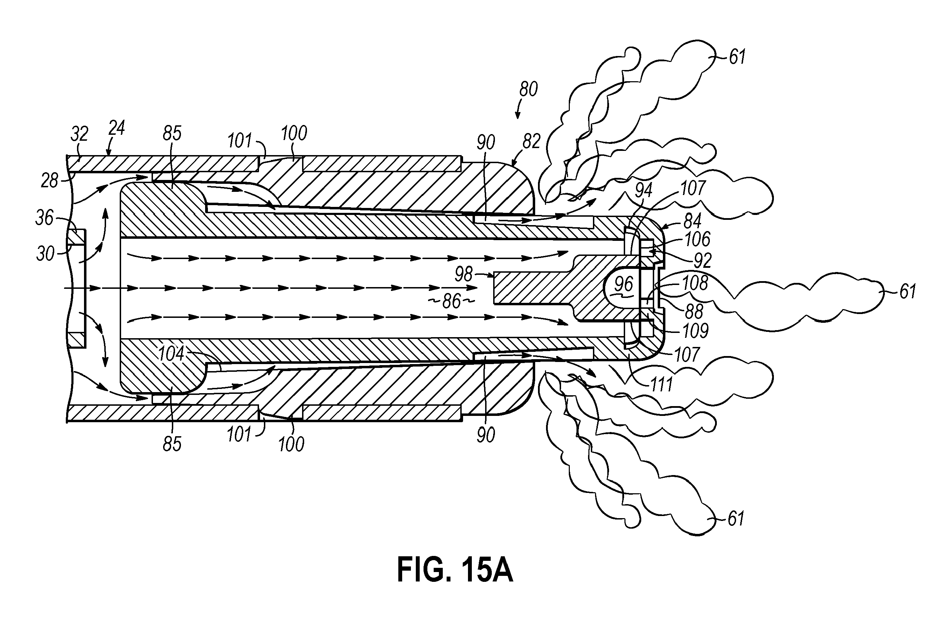

When the valve 84 contacts tissue of the surgical site 21, as shown in FIGS. 15 and 15A, the valve 84 is directed proximally into the tip housing 82 to the retracted position. With sufficient retraction of the valve 84, the release vents 90 enter into and form a fluid communication with the lumen 104 of the tip housing 82. Accordingly, fluid may flow along the first fluid path, as described above, or a second fluid path that extends from the inner and/or outer tubes 36, 32, into the lumen 104 of the tip housing 82, between the blades 85 of the valve 84, into a space between the tip housing 82 and the valve 84, and outwardly from the relief vents 90. Fluid flow along this second fluid path reduces the flow along the first fluid path. Yet, as was shown in FIG. 14A, when the valve 84 is in the extended position, the release vents 90 are positioned distal to the tip housing 82 and are not in fluid communication with the lumen 104 of the tip housing 82. Thus, when the valve 84 is in the extended position, only the first fluid path exists and all fluid flow is directed onto the surgical site 21.

FIG. 16 illustrates a spin chamber insert 124 in accordance with another embodiment of the present invention. Similar to the insert 98 of FIG. 13A, this spin chamber insert 124 includes a gas bubble 125 extending into a distal surface 126 and a plurality of cutouts 127 along the circumferential edge of the distal surface 126. This particular spin chamber insert 124 further includes an elongated proximal end having a stopper 128 thereon that is configured to at least partially block the inner tubing 36 (FIG. 3A).

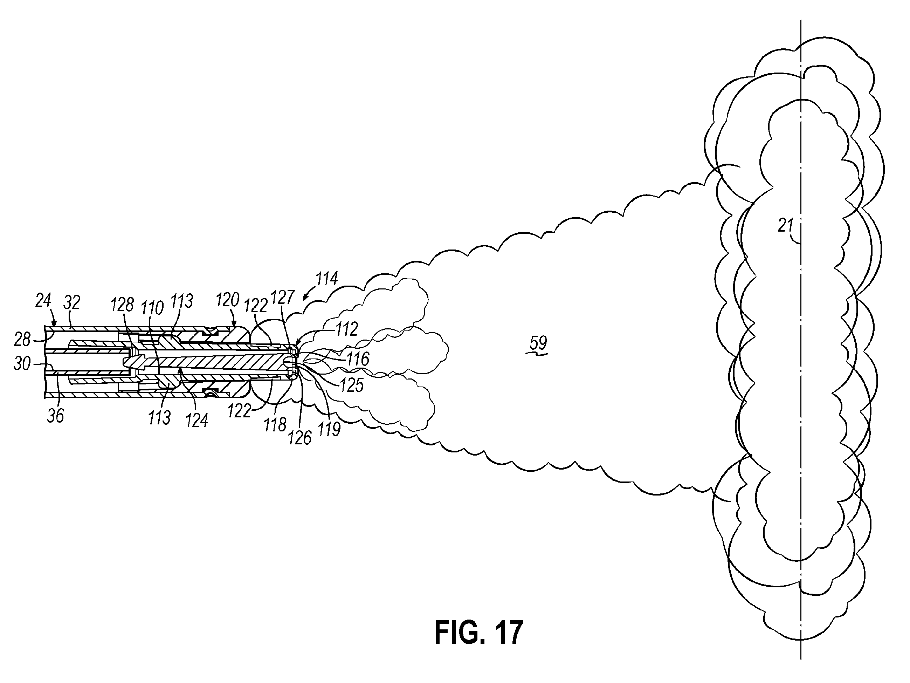

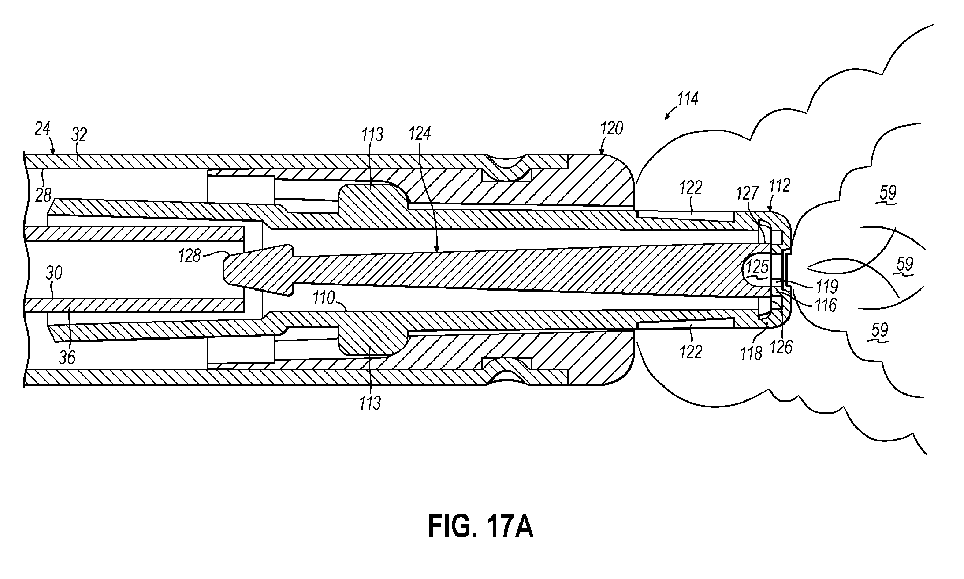

More particularly, FIGS. 17 and 17A illustrate a spray nozzle 114 having a tip housing 120 and a valve 112 with the spin chamber insert 124 of FIG. 16 incorporated therein. The tip housing 120, as shown, is similar to the tip housing 82 described in detail above with reference to FIG. 11. The valve 112 is similar to the valve 84 of FIG. 11; however, the lumen 110 of the valve 112 is extended such that it is directly and fluidically coupled to the inner tubing 36. Accordingly, the proximally-positioned stopper 128 is positioned proximate to, or may slightly enter into, the lumen 30 of the inner tubing 36. In this way, the inner tubing 36 may be operable as a spring that biases the spray nozzle 114 toward the extended position.

This particular, illustrative embodiment of the present invention, as shown in FIGS. 17 and 17A may be used in wet or dry applications. For example, when the valve 124 is in the extended position, a wet medicinal agent may be applied to the surgical site 21 via the first fluid path, which extends from the inner tubing 36, into the lumen 110 of the valve 112, between the inner and outer walls 116, 118 of the valve 112 by way of the cutouts 127 of the insert 124. The fluid then flows through the vanes 119 of the valve surface 126, into the bubble 125, and turns to be expelled from the spray nozzle 114 as the dispersion 59. In dry applications, the valve 124 moves into the retracted position such that the dry medicinal agent may be applied to the surgical site 21 via the second fluid path. As was described above, the second fluid path extends from the lumen 28 of the outer tubing 32, enters the lumen 110 of the tip housing 120 between the blades 113, between the outer surface of the valve 112 and the inner wall of the lumen 110, and is vented via the relief vents 122, which are in fluid communication with the lumen 110 of the tip housing 120.

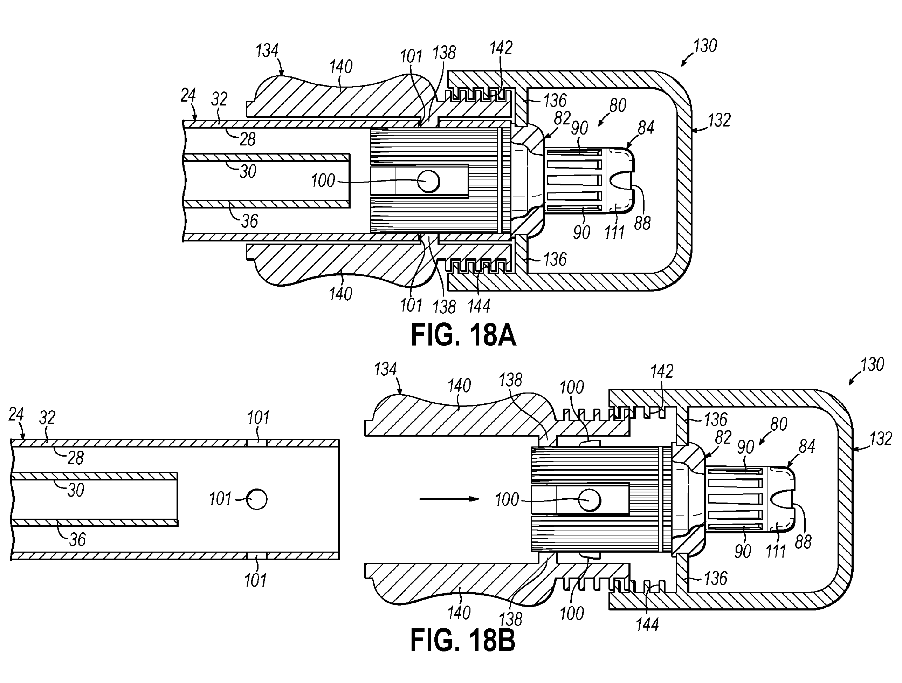

Turning now to FIG. 18, a tool 130 configured to remove the spray nozzle 80 from the multi-lumen tube 24 is shown in accordance with one embodiment of the present invention. Although the removal tool 130 is shown in use with the particular embodiment FIG. 8, it would be understood that this is for exemplary illustration only, and the tool 130 may be used with a spray nozzle in accordance with any embodiment of the present invention. The tool 130 includes a cover portion 132 configured to threadably engage a disengagement portion 134, which is configured to release the spray nozzle 80 from the multi-lumen tube 24. In that regard, the cover portion 132 may include a support 136 configured to engage and support the tip housing 82 during removal. The disengagement portion 134 includes arms 138 (two of which are shown) operably coupled to one or more finger pads 140 and in alignment with the snaps 100 provided on the outer surface of the tip housing 82. The user may actuate the arms 138, such as by pressing simultaneously and inwardly on the finger pads 140, e.g., with the forefinger and thumb, to direct the arms 138 inwardly. Because of the alignment between the arms 138 and the snaps 100, the snaps 100 are also compressed inwardly and released from the openings 101 of the multi-lumen tube 24. With the snaps 100 released, the cover portion 132 may be rotated, with respect to the disengagement portion 134, to uncouple the cover portion 132 from the disengagement portion 134. Further decoupling draws the spray nozzle 80 away from the multi-lumen tube 24. Still further rotation causes the spray nozzle 80 to be completely withdrawn from the multi-lumen tube 24 such that the spray nozzle 80 may be washed, sterilized, and/or disinfected, such as by ultrasonic cleaner or by an autoclave, for further use or otherwise disposed.

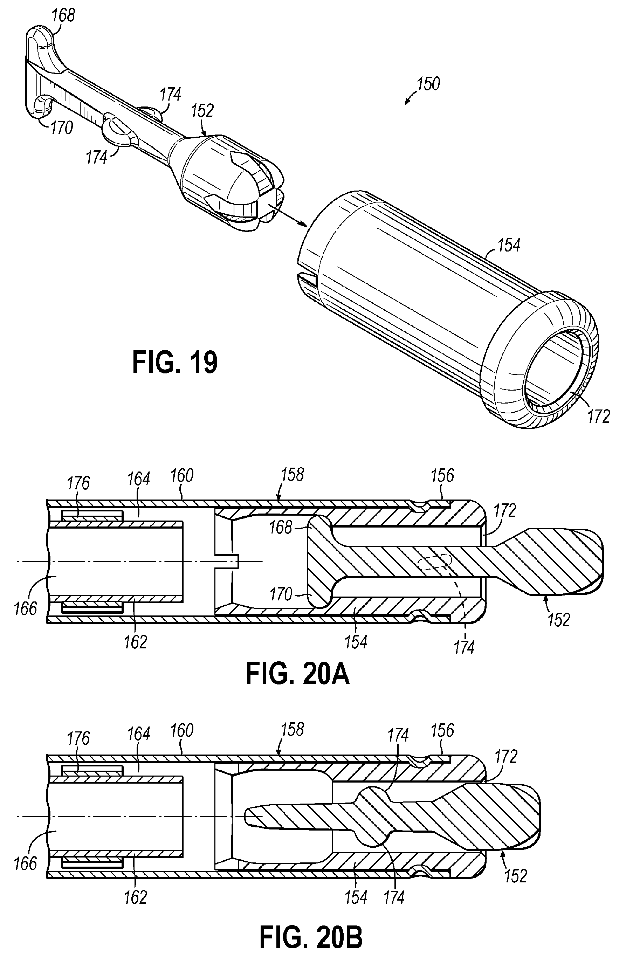

FIGS. 19, 20A and 20B illustrate an alternative spray nozzle 150 comprised of a valve 152 received in a tip housing 154, and operating similar to previously described embodiments. The tip housing 154 is secured within the distal end 156 of a multi-lumen tube 158 constructed generally similar to previously described embodiments, with differences to be described below. The multi-lumen tube 158 includes an outer tubing 160 and an inner tubing 162 spaced from the outer tubing 160 to create a gas lumen 164 and a powder lumen 166 which generally operate as previously described. In this embodiment, the valve 152 only includes two fins 168, 170 at its proximal end. The use of only two fins 168, 170 allows free flow of powder past the fins 168, 170 and out from the outlet 172 when the valve 152 is open (FIG. 20A). The valve 152 further includes two central locating members 174 to help center the valve 152 within the tip housing 154 as the valve 152 moves between the open position shown in FIG. 20A and the closed position shown in FIG. 20B. The tip housing 154 is crimped within the outer tubing 160.

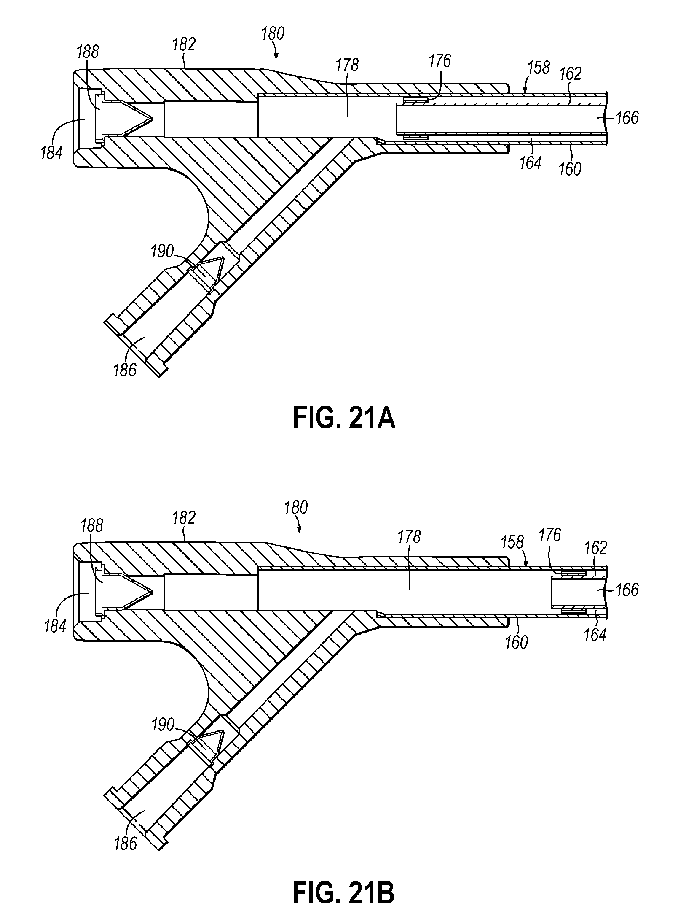

As further shown in FIGS. 21A and 21B, the inner tubing 162 may be secured in the outer tubing 160 using spacers 176, and adhesive, at a desired position to create a fluidizing chamber 178 at the proximal end of the applicator 180. The proximal end of the applicator 180 includes a hub 182 with a powder inlet 184 and a gas inlet 186. The inner tubing 162 is shown in two different positions in FIGS. 21A and 21B. The inner tubing 162 is secured in the desired position, such as by adhering the spacer 176, which is rigidly fastened to the inner tubing 162, to the inner wall of the outer tubing 160. The spacer 176 (FIG. 20A) at the distal end is similarly secured. In each position, a fluidizing chamber 178 is created proximal to the inner tubing 162. The fluidization chamber 178 in FIG. 21B is larger than that of FIG. 21A such that more fluidizing of the powder will occur prior to the powder entering the proximal end of the inner tubing 162. This feature allows the applicator 180 to be tailored to the density of the desired plume, and type(s) of powder to be used. The inlet lines 184, 186 have respective check valves 188, 190 which prevent backflow. The distal end of the applicator 180 will often be inserted into a gas insufflated area of a patient, such as the abdomen. The check valves 188, 190 will prevent insufflation gas from backing up into gas and powder supplies associated with the applicator 180. The moisture carried within the insufflation gas could create significant problems if it reached the powder supply, for example. The check valve 190 in the gas line further prevents back up of powder into the gas inlet 186.

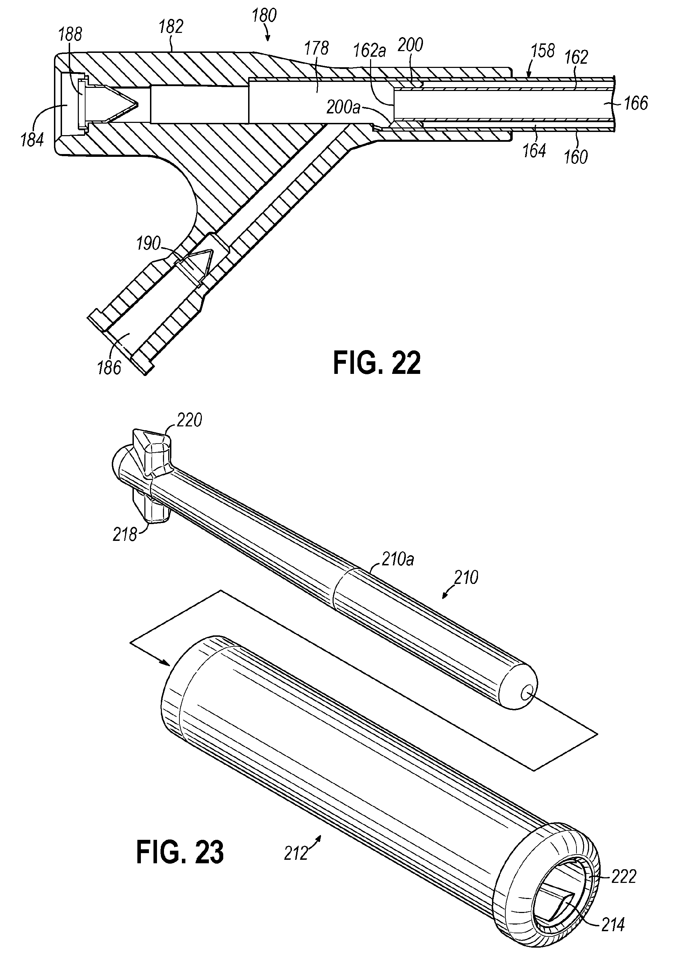

Fig. 22 illustrates an alternative hub configuration which is the same as shown in Figs. 21A and 21B except that the spacers 176 have been replaced by a cylindrical centering spacer 200. Cylindrical spacer 200 is affixed between outer tubing 160 and inner tubing 162, such as by using adhesive. The spacer 200 includes a bevel or converging portion 200a which is located adjacent the proximal end 162a of the inner tubing 162. This centering spacer 200 and particularly the bevel 200a is designed to help prevent the accumulation of powder at the proximal end of the inner tubing 162 and also help direct the powder into the powder lumen 166.

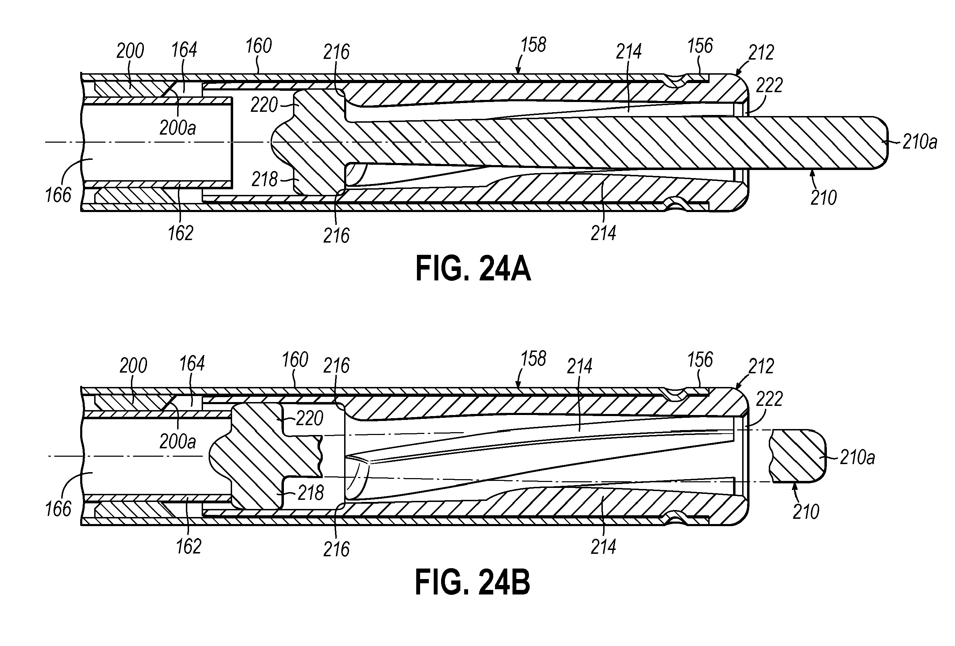

Figs. 23, 24A and 24B illustrate another alternative feature of the multi-lumen tube 158. In particular, a valve 210 is received in a tip housing 212 and operates similar to previously described embodiments. The difference is that the tip housing 212 incorporates mixing structure, such as in the form of generally spiral-shaped internal fins 214. The fin or other mixing structure 214 may be configured as shown and described in U.S. Patent Application Serial No. _(Atty. Ref.: NOR-1529US), filed on even date herewith and the disclosure of which is hereby incorporated by reference herein. The tip housing 212 includes internal stops 216 at the proximal end of the mixing structure 214. These stops 216 respectively engage two fins 218, 220 which act and serve generally the same purpose as the previously described fins 168, 170. The valve 210 moves between two positions as respectively shown in Figs. 24A and 24B for the same purposes as previously described. The stops 216 limit the distal travel of the valve 210. The distal end of the inner tubing 162 limits the proximal movement or travel of the valve 210. The valve 210 is in the open position as shown in Fig. 24A and in the closed and venting position as shown in Fig. 24B, generally as previously described with regard to earlier embodiments. When in the open position shown in Fig. 24A, powder and gas travel through the central powder lumen 166 and toward the distal end 156 of the multi-lumen tube 158. When the powder and gas travel through the section of the tube 158 that includes the mixing structure 214, the powder and gas are further mixed in a manner generally as described in the above-incorporated U.S. Patent Application Serial No. _(Atty. Ref.: NOR-1529US). Thus, the powder and gas travel generally between the stem 210a of the valve 210 and the generally spiral-shaped fins or other mixing structure 214 before exiting at the distal opening 222.

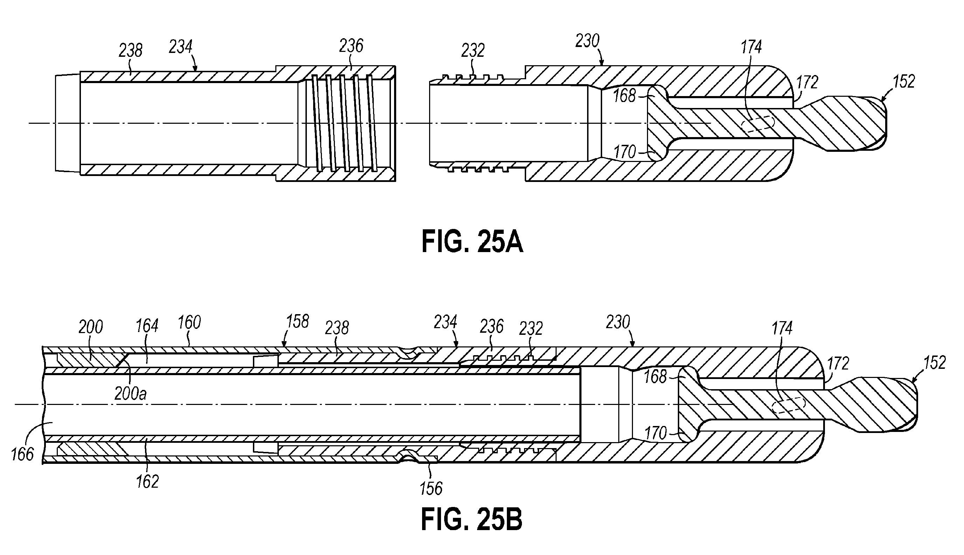

Figs. 25A and 25B illustrate another alternative feature incorporated generally at the distal end 156. In this embodiment, the distal end includes a replaceable tip 230 which may be designed and configured generally similar to embodiments as previously described such as by including a valve 152. The replaceable tip 230 includes a proximal end with a threaded element 232. An adapter 234 is provided with a mating threaded element 236. The adapter 234 includes a coupling portion 238 that is rigidly secured between the inner tubing 162 and the outer tubing 160, such as by using adhesive or welding techniques. Once the adapter 234 is secured in place, the distal end 156 effectively includes a threaded or other type of coupling element that may be selectively coupled with a mating element of the replaceable tip 230. In this embodiment, the distal end 156 will therefore include an internally threaded element 236 and the replaceable tip 230 includes a mating externally threaded element 232 allowing the replaceable tip 230 to be selectively coupled to and uncoupled from the distal end 156. This will, for example, allow different types of tips to be used on the same device and allow a tip to be replaced with a new tip for various purposes, such as if the tip becomes clogged with powder and/or other material such as biomaterials.

A spray nozzle for applying a medicinal agent to a tissue. The spray nozzle comprising a tip housing and a valve. The tip housing has a distal end, a proximal end, and a lumen that extends between the ends and defines a lengthwise central axis. The valve has an elongated body that extends through the lumen of the tip housing, along the lengthwise central axis. The valve, being configured to slide within the lumen of the tip housing, in an extended position with respect therewith is configured to disperse the medicinal agent toward the tissue. When the valve is in the retracted position with respect to the tip housing, the medicinal agent is vented away from the tissue.

While the invention has been illustrated by the description of embodiments thereof, and while the embodiments have been described in considerable detail, it is not intended to restrict or in any way limit the scope of the appended claims to such detail. Additional advantages and modifications will readily appear to those skilled in the art. Therefore, the invention in its broadest aspects is not limited to the specific details shown and described. The various features and embodiments disclosed herein may be used in any combination necessary or desired for a particular application. Consequently, departures may be made from the details described herein without departing from the spirit and scope of the claims which follow. What is claimed is:

## Claims

1. A spray nozzle for applying a medicinal agent to a tissue, the spray nozzle comprising:
a tip housing having a distal end, a proximal end, and a lumen extending therebetween and defining a lengthwise central axis; and
a valve having an elongated body extending through the lumen of the tip housing along the lengthwise central axis, the valve configured to slide within the lumen of the tip housing between an extended position and a retracted position,
wherein the tip housing with the valve in the extended position is configured to disperse the medicinal agent toward the tissue and the tip housing with the valve in the retracted position is configured to vent the medicinal agent away from the tissue.

2. The spray nozzle of claim 1, further comprising:
a plurality of relief vents fluidically coupled to the lumen of the tip housing and configured to vent the medicinal agent when the valve is in the retracted position.

3. The spray nozzle of claim 1, wherein the elongated body of the valve tapers proximally from a distally-positioned maximum width dimension.

4. The spray nozzle of claim 3, wherein an opening in the distal end of the tip housing has a width dimension that meets or exceeds the maximum width dimension of the valve such that when the valve is in the extended position, a clearance is formed between the elongated body of the valve and the opening of the distal end of the tip housing, and when the valve is in the retracted position, the maximum width dimension of the valve reduces the clearance.

5. The spray nozzle of claim 1, wherein the proximal end of the tip housing is configured to receive a tubing operable to supply the medicinal agent thereto.

6. The spray nozzle of claim 1, wherein the valve includes a plurality of vanes configured to alter a dispersion pattern of the medicinal agent.

7. The spray nozzle of claim 1, wherein the valve includes a proximal end, a distal end, and a lumen extending therethrough.

8. The spray nozzle of claim 7, wherein the distal end of the valve includes a spin chamber configured to mix the medicinal agent prior to dispersion toward the tissue.

9. The spray nozzle of claim 8, further comprising an insert within the lumen of the valve and proximate to the spin chamber, the insert having a gas bubble therein to receive the mixed medicinal agent from the spin chamber and redirect the mixed medicinal agent therefrom and toward the tissue.

10. The spray nozzle of claim 1, wherein the valve includes a plurality of relief vents formed on an outer surface of the elongated body, the plurality of relief vents configured to be in fluid communication with the lumen of the tip housing when the valve is in the retracted position and to provide a fluid path by which the medicinal agent is vented.

11. The spray nozzle of claim 1, wherein the tip housing further includes mixing structure configured to mix the medicinal agent prior to dispersion toward the tissue.

12. The spray nozzle of claim 1, wherein the tip housing further comprises a connector element configured to selectively couple with a distal end of an applicator tubing by a user.

13. A spray nozzle for applying a medicinal agent to a tissue, the spray nozzle comprising:
a tip housing having a distal end, a proximal end, and a lumen extending therebetween and defining a lengthwise central axis;
a valve having an elongated body with a proximal end, a distal end, and a lumen extending therethrough, the valve extending through the lumen of the tip housing along the lengthwise central axis and is configured to slide with respect thereto between an extended position and a retracted position;
a spin chamber at the distal end of the elongated body of the valve that is configured to mix the medicinal agent prior to dispersion toward the tissue;
a plurality of relief vents formed on an outer surface of the elongated body of the valve that is configured to be in fluid communication with the lumen of the tip housing when the valve is in the retracted position; and
an insert within the lumen of the valve and positioned proximate to the spin chamber, the insert having a gas bubble therein that is configured to receive the mixed medicinal agent from the spin chamber and to disperse the mixed medical agent,
wherein the tip housing with the valve in the extended position is configured to disperse the medicinal agent via the spin chamber and the insert toward the tissue and the tip housing with the valve in the retracted position is configured to vent the medicinal agent via the plurality of relief vents away from the tissue.

14. A method of applying first and second medicinal agents to a tissue using the spray applicator of claim 13 with a multi-lumen tubing operably coupled to the proximal end of the tip housing, wherein the lumen of the valve is operably coupled to a first lumen of the multi-lumen tubing and the lumen of the tip housing is operably coupled to a second lumen of the multi-lumen tubing, the method comprising:
with the valve in the extended position, directing the first medicinal agent from the first lumen of the multi-lumen tubing, through the lumen of the valve, through the spin chamber, into the gas bubble of the insert, and dispersing the first medicinal agent therefrom and toward the tissue; and
with the valve in the retracted position, directing the second medicinal agent from the second lumen of the multi-lumen tubing, through the lumen of the tip housing along an outer surface of the elongated body of the valve, into the plurality of relief vents of the valve, and venting the second medicinal agent therefrom and toward the tissue.

15. A method of applying a medicinal agent to a tissue using the spray nozzle of claim 13 with a tubing operably coupled to the proximal end of the tip housing, the method comprising:
with the valve in the extended position, directing the medicinal agent from the tubing, through the lumen of the valve, through the spin chamber, into the gas bubble of the insert, and dispersing the medicinal agent therefrom and toward the tissue; and
with the valve in the retracted position, directing the medicinal agent from the tubing, through the lumen of the tip housing along an outer surface of the elongated body of the valve, into the plurality of relief vents of the valve, and venting the medicinal agent therefrom and away from the tissue.

16. A spray applicator for applying a medicinal agent to a tissue, the spray applicator comprising:
a spray nozzle comprising:
a tip housing having a distal end, a proximal end, and a lumen extending therebetween and defining a lengthwise central axis; and
a valve having an elongated body extending through the lumen of the tip housing along the lengthwise central axis, the valve being configured to slide within the lumen of the tip housing between an extended position and a retracted position; and
a tubing operably coupled to the proximal end of the tip housing,
wherein the tip housing with the valve in the extended position is configured to disperse the medicinal agent toward the tissue and the tip housing with the valve in the retracted position is configured to vent the medicinal agent away from the tissue.

17. The spray applicator of claim 16, further comprising:
a plurality of relief vents fluidically coupled to the lumen of the tip housing and configured to vent the medicinal agent when the valve is in the retracted position.

18. The spray applicator of claim 16, wherein the elongated body of the valve tapers proximally from a distally-positioned maximum width dimension.

19. The spray applicator of claim 18, wherein an opening in the distal end of the tip housing has a width dimension that meets or exceeds the maximum width dimension of the valve such that when the valve is in the extended position, a clearance is formed between the elongated body of the valve and the opening of the distal end of the tip housing, and when the valve is in the retracted position, the maximum width dimension of the valve reduces the clearance.

20. The spray applicator of claim 16, wherein the valve includes a plurality of vanes configured to alter a dispersion pattern of the medicinal agent.

21. The spray applicator of claim 16, wherein the valve includes a proximal end, a distal end, and a lumen extending therethrough.

22. The spray applicator of claim 21, wherein the distal end of the valve includes a spin chamber configured to mix the medicinal agent prior to dispersion toward the tissue.

23. The spray applicator of claim 22, further comprising an insert within the lumen of the valve and proximate to the spin chamber, the insert having a gas bubble therein to receive the mixed medicinal agent from the spin chamber and redirect the mixed medicinal agent therefrom and toward the tissue.

24. The spray applicator of claim 21, wherein the tubing includes a first and second lumen therein, the lumen of the valve is operably coupled to the first lumen of the tubing being operably coupled to the lumen of the valve and the second lumen of the tubing being operably coupled to the lumen of the tip housing.

25. The spray applicator of claim 16, wherein the valve includes a plurality of relief vents formed on an outer surface of the elongated body, the plurality of relief vents configured to be in fluid communication with the lumen of the tip housing when the valve is in the retracted position and to provide a fluid path by which the medicinal agent is vented.

26. The spray applicator of claim 16, further comprising a hub operably coupled to an end of the tubing that opposes the end of the tubing having the spray nozzle operably coupled thereto, the hub configured to supply the medicinal agent to the tubing.

27. The spray nozzle of claim 16, wherein the tip housing further includes mixing structure configured to mix the medicinal agent prior to dispersion toward the tissue.

28. The spray nozzle of claim 16, wherein the proximal end of the tip housing further comprises a first connector element, and a distal end of the tubing includes a second connector element, wherein the first and second connector elements are configured to be selectively coupled together by a user.

29. A removal tool configured to remove the spray nozzle from the tubing of the spray applicator of claim 16, the removal tool comprising:
a cover portion configured to operably engage and support the tip housing; and
a disengagement portion configured to release the tip housing from the tubing,
wherein the cover portion is configured to move with respect to the disengagement portion such that when the disengagement portion releases the tip housing from the tubing, the cover portion, with the spray nozzle, is configured to move with respect to the disengagement portion and so as to withdraw the spray nozzle from the tubing.

30. The removal tool of claim 29, wherein an outer surface of the tip housing includes one or more snaps configured to secure the spray nozzle to the tubing, the disengagement portion having one or more arms configured to compress the one or more snaps into the tubing.

31. The removal tool of claim 29, wherein the cover portion threadably engages the disengagement portion.
